# EUROPEAN PATENT APPLICATION

(11) **EP 4 137 121 A1**
(43) Date of publication of application: **22.02.2023**
(21) Application number: 21191798.4
(22) Date of filing: 17.08.2021
(51) Int. Cl.: A61K 9/00, A61K 9/08, A61K 9/50, A61K 9/51, A61K 38/00

(54) **PHARMACEUTICAL COMPOSITIONS COMPRISING NANOPARTICLES FOR THE TARGETED DELIVERY OF ANTIGENS**

(71) Applicant: Topas Therapeutics GmbH, 20251 Hamburg (DE)
(72) Inventor: FLEISCHER, Sabine., 20251 Hamburg (DE); DIGIGOW, Reinaldo., 20251 Hamburg (DE); FANZUTTI, Marco., 20251 Hamburg (DE); MARQUES, Ligia Margarida., 20251 Hamburg (DE)
(74) Representative: Uexküll & Stolberg

(57) **Abstract**

The present invention provides pharmaceutical compositions comprising nanoparticles, wherein the nanoparticles each comprise
a) a micelle comprising an amphiphilic polymer, and
b) at least one peptide,
wherein the pharmaceutical composition comprises peptides comprising SEQ ID NO:1-4.

The present invention further provides respective pharmaceutical compositions for use in the treatment of Pemphigus vulgaris.

## Description

### FIELD OF THE INVENTION

The present invention provides pharmaceutical compositions comprising nanoparticles, wherein the nanoparticles each comprise a micelle comprising an amphiphilic polymer, and at least one peptide. The pharmaceutical composition of the present invention comprise peptides comprising SEQ ID NO:1-4. The pharmaceutical composition can be used in the treatment of Pemphigus vulgaris.

### BACKGROUND OF THE INVENTION

The liver plays a central role in the suppression of unwanted immune responses against blood-borne antigens, e.g. food antigens, entering the circulation. This fundamental mechanism of the liver can be employed to specifically downregulate detrimental immune responses against external protein antigens or autoantigens.

Antigen presentation by unconventional antigen-presenting cells (APCs), for example liver sinusoidal endothelial cells (LSECs), within the uniquely tolerogenic milieu of the liver is devised to maintain immune tolerance and homeostasis. This is achieved through various mechanisms of tolerance in the liver that encompass both cell-intrinsic and active regulatory mechanisms, including retention and deletion of activated T cells, and induction of regulatory T cells.

Ectopic expression of myelin-derived antigens in the liver can prevent autoimmune neuroinflammation in murine experimental autoimmune encephalomyelitis, an animal model for multiple sclerosis.

By targeting antigens to the liver for effective uptake and tolerogenic presentation, the extraordinarily effective scavenger functions of liver sinusoidal endothelial cells (LSECs) can be utilized to clear blood-borne antigens. For this purpose, antigenic peptides are conjugated to small (<200 nm) nanoparticles, designed to mimic blood-borne antigens. Like blood-borne antigens, these injected nanoparticle conjugates target the liver, where they are primarily taken up by LSECs. Upon intracellular uptake and processing, the peptides bind to MHC/HLA molecules and get presented at the cell surface where such peptide/MHC complexes are recognized by antigen-specific T cells. Within the tolerogenic milieu of the liver, this T cell-antigen recognition leads to T cell tolerance.

Using nanoparticles, each conjugated with a disease-related antigenic peptide it was shown in several different animal models, that both MHC/HLA-class I and II restricted peptides can induce tolerance in an antigen-specific way, thereby preventing or ameliorating diseases or unwanted immune responses.

Autoimmune diseases are representing a substantial burden for patients and healthcare systems. Current therapies mostly rely on immunosuppressive drugs, which however have considerable side effects. Autoantigen-specific immunotherapies that exclusively target disease-specific immunopathologies, leaving the patient's general immune status untouched, are considered an unmet medical need.

Pemphigus vulgaris (PV) is classified as a rare disorder and qualifies as a well-characterized CD4+ T cell-dependent autoantibody-mediated autoimmune disease. In human PV disease, autoreactive pathogenic autoantibodies, which are seen mainly in active stages of the disease, are the main pathological agent. They are predominantly directed against desmoglein-3, a cadherin which stabilizes the adhesion between epithelial keratinocytes. The interference of these autoantibodies with desmoglein-3 causes blisters due to loss of cell adhesion in mucosa and skin (termed akantholysis). The blisters associated with PV are fragile and easily ruptured, resulting in a severe loss of barrier function, infections and potentially fatal clinical outcome. Since autoreactive Dsg3-peptide-specific CD4+ T cells are considered to promote the autoimmune B cell response in PV resulting in the production of pathogenic anti-Dsg3 antibodies, it is aimed at therapeutically targeting Dsg3-peptide specific CD4+ T cells to ultimately reduce anti-Dsg3 antibody titers.

To this end, nanoparticles comprising an amphiphilic polymer shell can be used. The polymer shell forms a micellar structure with a surface structure allowing the covalent binding of the autoantigenic peptides.

WO 2013/072051 discloses a pharmaceutical composition for use in generating tolerized CD4+ T cells specific to at least one CD4+ T cell epitope in a subject for treating or preventing a disease wherein suppression of a specific immune response is beneficial. The nanoparticle comprises a micelle comprising an amphiphilic polymer and a peptide comprising at least one T cell epitope associated with the outside of the micelle. Nanoparticles, each carrying several copies of one specific peptide, can be mixed. This mixture of different nanoparticle-peptide conjugates is termed TPM (Topas particle mixture).

EP 20157797.0 relates to nanoparticles comprising a micelle comprising an amphiphilic polymer with a number average molecular weight (Mn) of 20,000 g/mol or less, and at least one peptide comprising at least one T cell epitope.

However, it was found that not all peptide nanoparticle combinations can be produced with a high density of peptides coupled to the surface of the nanoparticle. In certain medical applications it is important that the nanoparticles can be produced with a very high density of peptide loading, as well a high density of purity using efficient purification methods.

Thus, there is still a need in the art for improved pharmaceutical compositions which are suitable for use in human subjects. In particular, pharmaceutical compositions which induce a broad immune tolerance are needed.

### SUMMARY OF THE INVENTION

According to the present invention the problems described above, are solved by a pharmaceutical composition comprising nanoparticles, wherein the nanoparticles each comprise
a) a micelle comprising an amphiphilic polymer, and
b) at least one peptide,
wherein the pharmaceutical composition comprises peptides comprising SEQ ID NO:1-4.

The nanoparticles may further comprise a solid hydrophobic core which is at least partially coated by the polymer micelle or does not comprise a solid hydrophobic core.

The pharmaceutical composition of the present invention utilizes a natural liver-specific tolerance induction mechanism by employing a particle-mediated delivery method to target LSECs directly. The mix of at least four peptides, each coupled to the nanoparticles mimic innocuous food antigens, which are preferentially taken up by the LSECs after intravenous injection. LSECs present the peptides (after being cleaved off from the nanoparticles and bound to MHC/HLA molecules) to circulating desmoglein-3-specific CD4+ T cells resulting in their tolerization. In this way, peptide-specific immune tolerance can be induced for defined immune-disease-causing antigens, aiming at the amelioration of the disease. The inventors have surprisingly found that by using a mixture of nanoparticles, broader immune tolerance can be induced by several autoantigenic peptides at the same time.

In the humanized HLA-transgenic mouse model, immunization with human Dsg3 leads to the formation of Dsg3-specific IgG antibodies, which recognize the same spectrum of Dsg3-epitopes as IgG autoantibodies from PV patient sera. Injected into human epithelia in vitro, these antibodies induce pathologies comparable to PV.

A mixture of the four peptides with SEQ ID No: 1-4, each coupled to nanoparticles was proven to be effective in the HLA-transgenic humanized mouse model of PV by applying the mixture in a preventive mode, while a reduction to only three peptides in the mixture resulted in a significantly reduced tolerization effect.

It was demonstrated that a mixture of four peptides with SEQ ID No: 1-4, each coupled to nanoparticles more effectively reduces the formation of Dsg3-specific IgG in mice than a mixture of only three peptides, if administered before immunization with Dsg3, indicating that the mixture containing four Dsg3-peptides is superior to the mixture containing three Dsg3-peptides in inducing tolerance towards Dsg3.

### BRIEF DESCRIPTION OF THE FIGURES

Figure 1: Schematic structure of an exemplary nanoparticle
Figure 2: Flow chart of iron oleate complex synthesis; note that in Figure 2 (as well as in Figures 3-5, 7 and 8) the light blue arrows at the left side of the Figure indicate synthesis steps, whereas the darker blue arrows at the right hand of the Figure indicate purification steps
Figure 3: Flow chart of alternative iron oleate complex synthesis
Figure 4: Flow chart of SPIONs synthesis
Figure 5: Flow chart of LM-PMAOD synthesis
Figure 6: Flow chart of LM-PMAcOD synthesis
Figure 7: Flow chart of alternative LM-PMAcOD synthesis
Figure 8: Polymer coating of SPIONs
Figure 9: Flow chart of PMAcOD-SPION particle synthesis
Figure 10: Flow chart of peptide coupling and nanoparticle synthesis
Figure 11: Results of testing the treatment efficacy of three versus four TPC-Dsg3-peptide conjugates

### DETAILED DESCRIPTION OF THE INVENTION

According to the present invention, pharmaceutical compositions are provided which comprise nanoparticles. The nanoparticles each comprise a micelle comprising an amphiphilic polymer and at least one peptide. The pharmaceutical composition comprises peptides comprising SEQ ID NO:1-4. Thus, the pharmaceutical composition comprises peptides comprising the sequence of SEQ ID NO:1, SEQ ID NO:2, SEQ ID NO:3 and SEQ ID NO:4. SEQ ID NO:1-4 are characterized by the following amino acid sequences:
SEQ-ID 1: LNSKIAFKIVSQEPA,
SEQ-ID 2: TPMFLLSRNTGEVRT,
SEQ-ID 3: NIKVKDVNDNFPMFR, and
SEQ-ID 4: REGIAFRPASKTFTV.

According to the present application, the term "nanoparticle" is used interchangeably with "nanoscale particle". Such particles have a diameter of 1 to 999 nm, preferably, of 2 to 600 nm, 5 to 500 nm, 10 to 300 nm, 30 to 100 nm or 40 to 50 nm.

In the context of the present invention, a nanoparticle is a structure formed by at least a micelle and a peptide which is associated to the micelle. The peptides may either be associated to the outside of the micelle or encapsulated inside the micelle. The nanoparticles may further comprise a solid hydrophobic core which is at least partially coated by the micelle or does not comprise a solid hydrophobic core.

The pharmaceutical composition of the present invention comprises different types of nanoparticles, preferably at least four different types of nanoparticles. Each type may comprise at least one peptide sequence, which differs from the peptide sequence or peptide sequences of the other types of nanoparticles. Thus, the nanoparticles may differ in the peptide sequence. In a preferred embodiment, each type of nanoparticle comprises only one single type of peptide (having one specific amino acid sequence). Each nanoparticle can therefore comprise numerous peptides having the same amino acid sequence and the composition is obtainable by mixing different nanoparticles. An Investigational Medical Product (IMP) comprising a mixture of nanoparticles comprising a peptide consisting of SEQ ID NOs: 1-4 has been produced and tested.

In a preferred embodiment of the present invention, the pharmaceutical composition comprises between four and six different types of nanoparticles, wherein preferably all associated peptides of the different types of nanoparticles comprise at least one T cell epitope. In particular, each nanoparticle may be associated with a different antigenic peptide derived from desmoglein-3.

In a preferred embodiment of the present invention, the pharmaceutical composition comprises at least four different types of nanoparticles, wherein the different types of nanoparticles differ among each other in the peptide sequence, wherein a first type of nanoparticles comprises peptides consisting of SEQ ID NO:1, a second type of nanoparticles comprises peptides consisting of SEQ ID NO:2, a third type of nanoparticles comprises peptides consisting of SEQ ID NO:3 and a forth type of nanoparticles comprises peptides consisting of SEQ ID NO:4. Preferably, each type of nanoparticle comprises only peptides of the same peptide sequence.

In a particularly preferred embodiment of the present invention, the pharmaceutical composition comprises four different types of nanoparticles, wherein the different types of nanoparticles can be distinguished by their peptide sequence, wherein the first type of nanoparticles comprises peptides consisting of SEQ ID NO:1, the second type of nanoparticles comprises peptides consisting of SEQ ID NO:2, the third type of nanoparticles comprises peptides consisting of SEQ ID NO:3 and the forth type of nanoparticles comprises peptides consisting of SEQ ID NO:4. Preferably, each type of nanoparticle comprises only peptides of the same peptide sequence.

In an embodiment of the present invention, the pharmaceutical composition of the present invention comprises nanoparticles comprising a solid hydrophobic core, a micelle coating the core comprising an amphiphilic polymer with a number average molecular weight (Mn) of 20,000 g/mol or less, and at least one peptide. This has the additional advantage that the nanoparticles can be produced more easily and to a higher degree of purity. The inventors have surprisingly found that low molecular weight amphiphilic polymer generates fewer aggregates during coating of a solid core than a high molecular weight amphiphilic polymer. In addition, the low molecular weight amphiphilic polymer can be purified more efficiently compared to a high molecular weight amphiphilic polymer. In particular, unbound polymer can be separated more efficiently when a low molecular weight amphiphilic polymer is used in the nanoparticle of the invention.

In a preferred embodiment of the present invention, the nanoparticles in the pharmaceutical composition comprise
a) a micelle comprising an amphiphilic polymer comprising the following building block 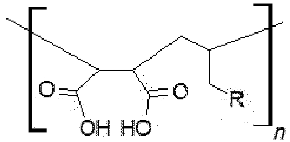 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) at least one peptide comprising one sequence of SEQ ID NO:1-4, wherein the peptide is covalently linked to the polymer, and
c) a solid hydrophobic core which is at least partially coated by the micelle, wherein the core comprises a traceable inorganic material selected from the group comprising iron oxide, CdSe/CdS/ZnS, silver and gold.

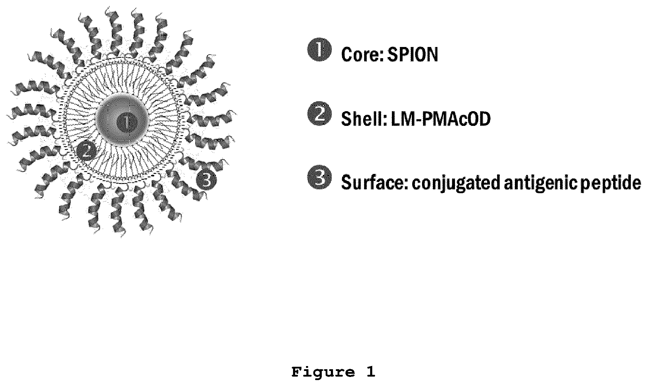

In a particularly preferred embodiment, all peptides covalently linked to the polymer of one nanoparticle b) have the same amino acid sequence and are covalently bound to the outside of a micellar structure comprising an amphiphilic polymer shell a) which consists of low molecular weight poly(maleic acid-alt-1-octadecene and a superparamagnetic iron oxide nanoparticle (SPION) core c) (schematic structure illustrated in Figure 1).

In a related aspect the present invention provides pharmaceutical compositions comprising at least four different types of nanoparticles, wherein the nanoparticles have a diameter of 10 to 300 nm and wherein each nanoparticle comprises
a) a micelle comprising or consisting of an amphiphilic polymer comprising the following building block 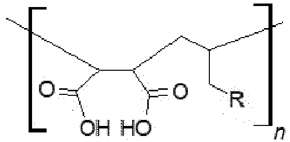 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) one peptide consisting of the sequence of one of SEQ ID NOs: 1-4, wherein the peptide is covalently linked to the polymer, and
c) a solid hydrophobic core which is at least partially coated by the micelle, wherein the core comprises a traceable inorganic material selected from the group comprising iron oxide, CdSe/CdS/ZnS, silver and gold.
wherein the four different types of nanoparticles differ among each other in the peptide sequence.

The above pharmaceutical compositions can be used for the treatment of *Pemphigus vulgaris,* in particular for the treatment of humans diagnosed with *Pemphigus vulgaris.*

The present invention thus provides pharmaceutical compositions for the treatment of *Pemphigus vulgaris,* which compositions comprise at least four different types of nanoparticles, wherein the nanoparticles have a diameter of 10 to 300 nm and wherein each nanoparticle comprises
a) a micelle comprising or consisting of an amphiphilic polymer comprising the following building block 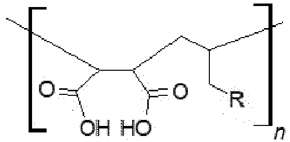 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) one peptide consisting of the sequence of one of SEQ ID NOs: 1-4, wherein the peptide is covalently linked to the polymer, and
c) a solid hydrophobic core which is at least partially coated by the micelle, wherein the core comprises a traceable inorganic material selected from the group comprising iron oxide, CdSe/CdS/ZnS, silver and gold.
wherein the four different types of nanoparticles differ among each other in the peptide sequence.

### The nanoparticles

The nanoparticles in the pharmaceutical composition of the present invention may additionally comprise a moiety, e.g., a carbohydrate or a protein targeting them, or enhancing targeting to specific cells such as liver sinusoidal endothelial cells and/or Kupffer cells. Such moiety could, e.g., enhance or accelerate uptake from the circulation via receptor mediated endocytosis. Examples of suitable modifications are carbohydrates such as mannose.

The nanoparticle, by virtue of the polymer forming the micelle, may be negatively charged or uncharged; preferably, the nanoparticle is negatively charged at a pH of 6 to 7 (pH during measurement). The polymer coating may comprise acid, e.g., carboxylic acid groups, leading to a negative charge of the nanoparticle.

The nanoparticles used in the pharmaceutical composition of the present invention may have a zeta potential between -20 and -60 mV, preferably between -30 and -50 mV, more preferably between -35 and -45 mV at pH 6 to 7 (pH during measurement) . The zeta potential can be measured using a Malvern Zetasizer Nano ZS instrument.

The nanoparticles may have a hydrodynamic diameter (z-average) between 10 and 100 nm or 10 and 70, preferably between 10 and 50, more preferably between 15 and 40 m, most preferably between 20 and 35 nm, as measured by dynamic light scattering (DLS) .

The nanoparticles may have a polydispersity index below 0.50, preferably between 0.05 and 0.45, more preferably between 0.10 and 0.40, as measured by dynamic light scattering (DLS).

The determination of the hydrodynamic diameter and the polydispersity index is carried out using electrophoretic light scattering analysis methods, preferably a Malvern Zetasizer. In one embodiment the method for determining the hydrodynamic diameter and the polydispersity index is carried out using electrophoretic light scattering, disposable polystyrene cuvettes, Zetasizer Software 7.12, milli-Q water. The nanosphere size standards of 20 nm and 100 nm (NIST certified or equivalent) are diluted in an aqueous 0.9% sodium chloride solution and the test samples are diluted in water. All aqueous reagents are filtered through 0.22 µm membrane prior to use. In the most preferred embodiment of the invention, the method for determining the hydrodynamic diameter and the polydispersity index is carried out using electrophoretic light scattering in combination with the following analysis conditions:

**Overview of the analysis conditions:**

| **Parameter** | **Setting** |
|---|---|
| Dispersant name | Water |
| Dispersant RI | 1.33 |
| Viscosity (cP at 25,0 °C) | 0.8872 |
| Material RI (sample) | 2.42 |
| Material RI (standards) | 1.333 |
| Material Absorption | 0.05 |
| Temperature (°C) | 25 |
| Measurement Position (mm) | 4.65 |
| Cell description | Disposable sizing cuvette |
| Attenuator | Auto |
| Measurement duration | Auto |

The evaluation of the data is based on mean diameter (Z-Average, nm by intensity), which is a parameter also known in DLS as the cumulants mean and Polydispersity index (PDI), which is used as a measure of the size distribution.

Furthermore, the nanoparticles in the pharmaceutical composition of the present invention may have a total polymer content of 0.1 to 5 mg/mL, preferably 0.5 to 4 mg/mL, more preferably of 1 to 3 mg/mL. The total polymer content is determined by GPC. For measuring the total polymer content, the peptides are hydrolyzed, and the particles are destroyed (e.g using a 6 M HCl solution). The polymer is extracted after addition of EDTA. After evaporation of solvent, the residue is re-dissolved and the polymer content is determined by GPC.

The determination of total polymer content is preferably carried out using the following reagents and reference standards: water (HPLC grade), acetonitrile (HPLC grade), tetrahydrofuran with BHT (THF-HPLC grade), acetic acid 100% (analytical grade), hydrochloric acid 37% (analytical grade), ethylenediaminetetraacetic acid disodium salt dihydrate (analytical grade), ethyl acetate (analytical grade), sodium hydroxide (analytical grade) and poly(maleic acid-alt-1-octadecene) as reference material. The chromatographic conditions for the determination of total polymer content are:

| | |
|---|---|
| Column | Agilent PL-gel Mixed-D, 300+75 mm ID, 5µm |
| Column temperature | 40°C |
| Flow rate | 1.0 mL/min |
| Detector | Refractive index detector at 35°C |
| Sample rating | 2.31 Hz or equivalent |
| Injection volume | 20 µL |
| Autosampler temperature | Ambient |
| Run time | 15 min |
| Mobile phase | THF/Acetic acid [90/10]%(v/v) |
| Mobile phase program | Isocratic |

The nanoparticles in the pharmaceutical composition of the present invention comprise a high amount of peptides. In particular, the pharmaceutical composition may have a total peptide content of more than 0.1 mM, preferably 0.2 to 1 mM, more preferably of 0.3 to 0.8 mM. The total peptide content can be measured by hydrolyzing the peptides with HCl and derivatizing the resulting amino acids with ortho-phthaldehyde prior to HPLC analysis. The samples can be analyzed by standard addition with amino acid standard solution.

The different components of the nanoparticles used in the pharmaceutical composition of the present invention are described in more detail in the following sections.

### The micelle

In the context of the present invention, the term "micelle" relates to an aggregate of amphiphilic molecules dispersed in an aqueous solution. The hydrophilic parts of the amphiphilic molecules are in contact with the surrounding solvent, sequestering the hydrophobic "tail" regions of the amphiphilic molecules on the inside of the micelle, thus providing a dissolution like distribution behavior of the nanoparticles in aqueous liquids, i.e. render the nanoparticles water-soluble. This type of micelle is also known as a normal phase micelle (or oil-in-water micelle).

The micelle can be formed by one, but also by more than 1, e.g., 2, 3 or 4 amphiphilic polymeric molecules. The micelle can be formed by the same or by different amphiphilic polymeric molecules. In general, in the context of the specification, "a" or "the" is not intended to be limiting to "one" unless specifically stated.

In a preferred embodiment, the micelle is formed by a single layer of amphiphilic polymers.

Such a micelle can be structurally distinct from a bilayer or a liposome formed by an amphiphilic polymer. In this case the structures are not, or not to a significant percentage (e.g. not more than 10%, more than 5%, or preferably, more than 1%), comprised in the nanoparticle of the present invention.

In one embodiment of the present invention, the amphiphilic polymer is used to produce at least 70%, preferably at least 90% of the micelle. In a preferred embodiment, the micelle consists of the amphiphilic polymer.

In some embodiments of the present invention the nanoparticles do not comprise a solid hydrophobic core. In other embodiments, the nanoparticles comprise the micelle and a solid hydrophobic core.

In one embodiment of the present invention, the micelle may be co-stabilized with further components such as fatty acids or phosphatidylcholines. In this regard, preferred fatty acids are stearic acid or oleic acid. A preferred phosphatidylcholine is selected from Lipoid S100, Lipoid S PC3 and DSPC. Cholesterol may also be used as a co-stabilizer.

### The amphiphilic polymer

The amphiphilic polymer generally comprises a hydrophobic region comprising a hydrophobic aliphatic chain having a length of 8 to 23, preferably 8 to 21, most preferably 16 to 18 carbon atoms. The hydrophilic region of the amphiphilic polymer may be negatively charged in an aqueous solution.

In a preferred embodiment of the present invention, the amphiphilic polymer spontaneously forms micelles in solution. When a solid hydrophobic core is present, the amphiphilic polymer forms micelles around the solid core, rendering the nanoparticle water-soluble.

The number average molecular weight (Mn) of the amphiphilic polymer can be 20,000 g/mol or less, preferably 10,000 g/mol or less, or 6,000 g/mol or less, more preferably from 6,000 to 1,000 g/mol, most preferably from 3,000 to 6,000 g/mol.

The number average molecular weight may be determined using gel permeation chromatography (GPC), preferably using polystyrene as calibration standard.

In a preferred embodiment the number average molecular weight is determined using a PL-gel mixed D column at a temperature of 40°C, a mobile phase consisting of tetrahydrofuran/acetic acid 90/10% (v/v), a flow rate of 1.0 ml/min, in combination with a refractive index detector at a temperature of 35°C and polystyrene as calibration standard.

In the most preferred embodiment, the determination of the number average molecular weight uses GPC and the following measurement conditions:

| Reference standards | Polystyrene standard (MW (nominal Mp); 1000 g/mol to 130000 g/mol |
|---|---|
| Column | Agilent PL-gel mixed-D, 300 x 7.5 mm ID, 5 µm |
| Column Temperature | 40°C |
| Detector | Refractive index detector at 35°C |
| Flow rate | 1.0 ml/min |
| Injection volume | 20 µL |
| Autosampler temperature | Ambient |
| Run time | 15 min |
| Mobile phase | Tetrahydrofuran/Acetic acid [90/10]%(v/v) |
| Mobile phase program | Isocratic |

The amphiphilic polymer may be an alternating copolymer. An alternating copolymer is a copolymer comprising two species of monomeric units distributed in alternating sequence.

In one embodiment of the present invention, the amphiphilic polymer is a copolymer of maleic anhydride and at least one alkene.

The alkene used in the production of the amphiphilic polymer may be selected from one or more of 1-decene, 1-undecene, 1-dodecene, 1-tridecene, 1-tetradecene, 1-pentadecene, 1-hexadecene, 1-heptadecene, 1-octadecene, 1-nonadecene or 1-eicosene, preferably the alkene is 1-octadecene.

In a preferred embodiment of the present invention, the amphiphilic polymer is a copolymer of maleic anhydride and an alkene.

In a preferred embodiment of the present invention, the amphiphilic polymer has a main hydrophilic poly-maleic anhydride backbone having hydrophobic alkyl side chains. Typically, the side chain can have from 5 to 23 carbon atoms, in particular from 9 to 21 atoms. In a most preferred embodiment, the side chains are linear and have from 10 to 18 carbon atoms.

The amphiphilic polymer may comprise the following building block 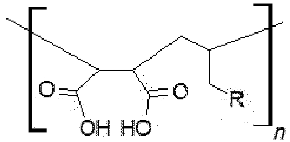 wherein R is a hydrocarbyl group or a substituted hydrocarbyl group. In a preferred embodiment of the present invention, R is a C₄ to C₂₂ alkyl group, such as a C₇ to C₁₉ alkyl group.

In an even more preferred embodiment, R is a linear alkyl group, preferably a linear C₇ to C₁₇ alkyl group, most preferably R is a linear pentadecyl group or a linear nonyl group.

The amphiphilic polymer may consist of the building block defined above.

In other embodiments according to the present invention, the amphiphilic polymer comprises at least 50 %, preferably at least 70 %, most preferably more than 90 % of the building block defined above.

In a preferred embodiment, the amphiphilic polymer is selected from the group comprising poly(maleic acid-1-octadecene), poly(maleic acid-1-tetradecene) or poly(maleic acid-1-dodecene), preferably the polymer is poly(maleic acid-1-octadecene) and the number average molecular weight of the polymer is from 6,000 to 1,000 g/mol.

In a specifically preferred embodiment, the amphiphilic polymer is selected from the group comprising poly(maleic acid-*alt*-1-octadecene), poly(maleic acid-*alt*-1-dodecene) and poly(maleic acid-*alt*-1-tetradecene), preferably the polymer is poly(maleic acid-*alt*-1-octadecene) and the number average molecular weight of the polymer is from 5000 to 1000 g/mol.

### The peptides

The pharmaceutical composition of the present invention comprises peptides comprising SEQ ID NO:1-4 which form part of the nanoparticles.
SEQ-ID 1: H₂N-LNSKIAFKIVSQEPA-COOH,
SEQ-ID 2: H₂N-TPMFLLSRNTGEVRT-COOH,
SEQ-ID 3: H₂N-NIKVKDVNDNFPMFR-COOH, and
SEQ-ID 4: H₂N-REGIAFRPASKTFTV-COOH.

In a preferred embodiment, the at least one peptide consists of one of the sequence of SEQ ID NO:1-4. In another embodiment, the at least one peptide comprises not more than 2, preferably not more than 1 additional amino acid (in addition to the sequence of SEQ ID NO:1-4).

Preferably, the pharmaceutical composition of the present invention comprises only peptides having sequences SEQ ID NO:1-4. This means, that in this embodiment, only these peptides may be present, but this does not exclude the presence of other components in the composition.

The peptide may be synthesized, recombinantly expressed or isolated or modified from natural sources.

The peptide may be associated with the outside of the micelle or encapsulated in the inside of the micelle (in embodiments where no solid hydrophobic core is present in the nanoparticle). Accordingly, the peptide may be localized on the outside of the micelle or inside the micelle.

The peptide may be covalently linked to the micelle or non-covalently associated, preferably covalently linked to the micelle.

In a preferred embodiment, the peptide is covalently linked to the micelle using a method of covalently coupling peptides known in the art such as carbodiimide or succinimide coupling. Preferably, the peptide is covalently linked to the micelles using 1-Ethyl-3-(3-dimethylaminopropyl)carbodiimide (EDC) chemistry.

### The solid hydrophobic core

In one embodiment of the present invention the nanoparticles comprise a solid hydrophobic core which is at least partially coated by the micelle. The core can be an inorganic core, preferably comprising iron oxide, CdSe, silver or gold.

The diameter of the core may be 2 to 500 nm, preferably, 3 to 25 nm, more preferably, 5 to 15 nm. The diameter of the core may be determined using transmission electron microscopy (TEM) or small-angle X-ray scattering (SAXS).

Exemplary inorganic cores are iron oxide nanoparticles stabilized by oleic acid or another carboxylic acid (C₁₄-C₂₂, preferably, C₁₆-C₁₈), quantum dots (CdSe/CdS/ZnS stabilized, e.g., by trioctyloxinphosphinoxide), gold nanoparticles, e.g., stabilized by sulfonic compounds.

Such inorganic cores by themselves are typically not stable in an aqueous solvent such as water, but embedding them in the polymeric micelles renders them water-soluble. The hydrophobic parts of the amphiphilic polymer interact with the hydrophobic core of the nanoparticle, leading to the formation of a single coating layer of polymer surrounding the core. In the coating process the amphiphilic polymer can replace the hydrophobic part of the core by ligand exchange and the double layer micelle is thus formed around the core. In one embodiment of the invention, the polymer at least partially replaces the oleic acid on the surface of the core particle and the hydrophilic part of the polymer interacts with the surface of the iron oxide core and the hydrophobic part of the polymer interact with each other forming a double layer micelle around the iron oxide core, resulting in an iron oxide coated with polymer.

According to a preferred embodiment of the present invention, the core is superparamagnetic.

In a specifically preferred embodiment of the present invention, the core is a superparamagnetic iron oxide nanoparticle (SPION), which may be stabilized by oleic acid.

The cores preferably render the nanoparticles of the invention traceable, e.g., by their characteristics in fluorescence, electron microscopy or other detection method.

### Production of the nanoparticles

The nanoparticles present in the pharmaceutical composition of the present invention may be produced by a method comprising the following steps:
a) obtaining a hydrophobic core nanoparticle,
b) obtaining an amphiphilic polymer, preferably using radical copolymerization,
c) optionally purifying the amphiphilic polymer,
d) mixing of the hydrophobic core nanoparticles and the amphiphilic polymer to form micelles,
e) adding at least one peptide to form the nanoparticles.

If the peptides are encapsulated by the micelle, step e) is performed prior to step d). In this case, the peptides are added to the amphiphilic polymer prior to micelle formation.

The hydrophobic core of step a) can be synthesized using appropriate reactants in solution. Preferably, the hydrophobic core is synthesized using metal salts and salts of carboxylic acids as reactants in the presence of organic solvents. Preferably, the reaction is conducted at elevated temperatures under oxygen restriction.

The amphiphilic polymer used in the nanoparticles of the present invention may be prepared by a radical copolymerization using a radical initiator.

One method of obtaining the amphiphilic polymer with a number average molecular weight (Mn) of 20,000 g/mol or less resides in synthesizing the same using a two step method, comprising a step of producing a polymer of the anhydride and a step of hydrolyzing the anhydride to obtain an acid. The molecular weight of the polymer can be controlled by varying the concentrations of the reactants or the amount of radical initiator. The molecular weight of the polymer can be analyzed by gel permeation chromatography. The copolymerization may be conducted in an organic solvent such as 1,4 dioxane, xylene or chlorobenzene.

Many radical initiators are known in the art; they include various peroxides and azo-type compounds. Examples of suitable peroxides are benzoyl peroxide, lauryl peroxide, di-t-butyl peroxide, 2,4-dichlorobenzyl peroxide, t-butyl hydroperoxide, cumene hydroperoxide, diacetyl peroxide, diethyl peroxycarbonate, t-butyl perbenzoate and perborates. Suitable azo-type compounds include 2,2'-Azobis(2-methylpropionitrile), p-bromobenzenediazonium fluoborate, p-tolyldiazoaminobenzene, p-bromobenzenediazonium hydroxide, azomethane and phenyldiazonium halides. Preferably, the radical initiator is 2,2'-Azobis(2-methylpropionitrile).

The copolymerization may be conducted at elevated temperatures such as from 70 to 120°C, preferably from 90 to 110°C. Preferably, the copolymerization is initiated by heating the mixture to 70 to 120°C, preferably from 90 to 110°C.

Step b) may comprise the steps of mixing the reactants, deoxygenizing the mixture, heating the mixture and then cooling the mixture. Afterwards, the polymer may be dissolved and stirred overnight. The formed solid may be recovered, preferably using centrifugation.

Step b) may include the addition of a base to the polymer (*e.g.* NaOH). Preferably, the base is reacted with the polymer at elevated temperature, preferably between 50°C and 70°C, such as 60°C until almost all solids is dissolved. The resulting suspension may be acidified (*e.g.* pH <2). Afterwards, the reaction mixture may be extracted with an organic solvent such as ethyl acetate. The organic layer may be extracted with a sodium hydroxide solution. The aqueous solution may be again extracted with an organic solvent such as ethyl acetate and then dried to obtain the purified amphiphilic polymer.

The polymer may be further purified (step c). Preferably, the polymer is further purified by extracting the polymer with n-hexane or n-heptane. The extraction can be performed at concentrations of greater than 10 g/L, preferably 100 g/l. Furthermore, an additional purification step of the amphiphilic polymer may be added. In this additional purification step, the crude reaction product of the polymerization is dissolved and precipitated. In a preferred embodiment, the solvent is dichloromethane and the polymer is precipitated using a mixture of methanol/heptane or acetonitrile/iso-propanol. The mixtures used may contain for example 95/5% (v/v%) methanol/heptane, 10/90 (v/v%) acetonitrile/iso-propanol or 5/95 (v/v%) acetonitrile/isopropanol. In a preferred embodiment, the precipitation mixture is added at temperatures of -10 to 10°C, preferably -5 to 5°C.

The purity of the amphiphilic polymer after hydrolysis and workup can be measured by ¹H NMR.

The micelle may be formed (step d)) by forming a solution containing the amphiphilic polymer. Preferably, the micelle is formed in an aqueous solution. Co-stabilizers may be added to the amphiphilic polymer to improve micelle formation. Preferably, step d) comprises the sub-steps of solubilising the amphiphilic polymer and the core particles, removing the solvent until a thin film is formed, adding a basic aqueous solution at increased temperature and ambient pressure to form an aqueous colloidal dispersion, diluting the solution and optionally filtering it. Afterwards, several washing steps may be applied.

The peptides to be used in step e) may be synthesized using state of the art solid phase chemistry.

The synthesis of the peptides can be accomplished via Fmoc chemistry from the C to N direction using solid phase peptide synthesis (SPPS). The alpha amino group of each amino acid is protected with a fluoren-9-ylmethoxycarbonyl (Fmoc) group, while side chain functional groups are also blocked with various appropriate protective groups. In general, the SPPS consists of repeated cycles of N-terminal deprotection followed by coupling reactions. The first Fmoc-protected amino acid is coupled to the resin. Afterwards, the amine group is deprotected with a mixture of piperidine in dimethylformamide (DMF), and then coupled with the free acid of the second Fmoc-protected amino acid. The cycle is repeated until the desired sequence is obtained. The resin is washed between each step. The completion of each coupling reaction is monitored by a qualitative ninhydrin test. In the last step of the synthesis, the crude peptide-resin is successively washed with DMF and methanol, and dried. Then, the protective groups are removed from the peptide and the peptide is cleaved from the resin using trifluoroacetic acid (TFA). The obtained crude peptide is isolated by ether precipitation from the cleavage mixture. Further, the peptide is purified through preparative HPLC to reach purity requirements, and the counter ion TFA is replaced with chloride by using an appropriate solvent-buffer system. Finally, the purified peptide is lyophilized.

The resulting nanoparticles may be purified using intensive washing and filtration steps to remove the coupling reagent(s) and any low molecular weight components.

### The composition

The pharmaceutical composition of the present invention may comprise a liquid or lyophilized carrier. It is evident that, in particular for administration to a human subject, the composition preferably is sterile and biologically compatible.

In a preferred embodiment, the pharmaceutical composition comprises nanoparticles in a liquid carrier. The liquid carrier is preferably water or water-based, e.g., a aqueous buffer such as Phosphate buffered saline (PBS), Ringer solution, TRIS buffer or sodium chloride solution. Suitable preservatives may or may not be contained.

In a preferred embodiment, the pharmaceutical composition of the present invention comprises the nanoparticles dispersed in an aqueous buffer, wherein the buffer preferably comprises at least one sugar, at least one primary amine and/or at least one aminoacid. In a particularly preferred embodiment, the composition comprises the nanoparticles dispersed in an aqueous solution of D-mannitol, Tris(hydroxymethyl)aminomethan (TRIS) and/or L-lactic acid. The use of this buffer has the advantage that the particles are very stable in this buffer and can be lyophilized later on.

The pharmaceutical composition of the present invention may comprise at least 4 different types of nanoparticles. Preferably, each type comprises at least one peptide sequence, which differs from the peptide sequence or peptide sequences of the other types of nanoparticles. The pharmaceutical composition may comprise each type of nanoparticle in a concentration below 100 µM, preferably from 0.5 to 80 µM, most preferably from 1 to 50 µM. The pharmaceutical composition of the present invention may comprise the different types of nanoparticles in equimolar concentration.

In a preferred embodiment of the present invention, the pharmaceutical composition of the present invention comprises 4 different types of nanoparticles in equimolar concentration, preferably dispersed in an aqueous buffer.

In a preferred embodiment of the present invention, the pharmaceutical composition of the present invention comprises nanoparticles comprising
a) a micelle comprising an amphiphilic polymer comprising the following building block 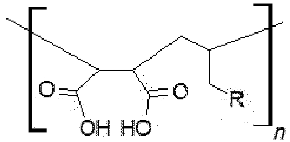 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) at least one peptide comprising the sequence of one of SEQ ID NO:1-4; and
c) a solid hydrophobic core which is at least partially coated by the micelle, wherein the core comprises a traceable inorganic material selected from the group comprising iron oxide, CdSe/CdS/ZnS, silver and gold.

In a preferred embodiment, the pharmaceutical composition comprises 4 different types of nanoparticles, wherein each nanoparticle comprises
a) a micelle comprising an amphiphilic polymer comprising the following building block 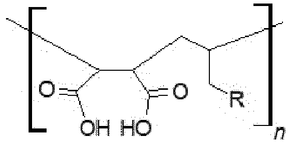 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) one peptide selected from the sequence of SEQ ID NO:1-4; and
c) a solid hydrophobic core which is at least partially coated by the micelle, wherein the core comprises a traceable inorganic material selected from the group comprising iron oxide, CdSe/CdS/ZnS, silver and gold; and
wherein the 4 different types of nanoparticles differ among each other in the peptide sequence, wherein the first type of nanoparticles comprises peptides having SEQ ID NO:1, the second type of nanoparticles comprises peptides having SEQ ID NO:2, the third type of nanoparticles comprises peptides having SEQ ID NO:3 and the fourth type of nanoparticles comprises peptides having SEQ ID NO:4.

In a particulary preferred embodiment, the pharmaceutical composition comprises 4 different types of nanoparticles, wherein each nanoparticle consists of
a) a micelle comprising an amphiphilic polymer comprising the following building block 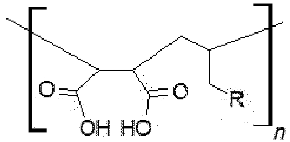 wherein R is a hydrocarbyl group or a substituted hydrocarbyl group, preferably R is a linear alkyl group, preferably a linear C₁₁ to C₁₇ alkyl group, and wherein the polymer has a number average molecular weight (Mn) of 6,000 to 1,000 g/mol, and
b) numerous peptides having the same amino acid sequence, which sequence is selected from SEQ ID NO:1-4; and
c) a solid iron oxide core.

### The use of the composition

The pharmaceutical composition of the present invention can be used in the treatment of Pemphigus vulgaris. According to the present invention, the term "treating" is used to refer to the alleviation of symptoms of a particular disease in a subject, and/or improvement of an ascertainable measurement associated with a particular disorder. The present invention provides a composition for use in inducing immunological tolerance to Pemphigus vulgaris autoantigens.

The pharmaceutical compositions may be administered to a subject in need thereof.

The required dose and concentration for administration to the subject may be determined by the responsible medical attendant according to the facts and circumstances of the case. An exemplary dose might comprise 0.03 µmol to 0.90 µmol per patient body weight, e.g., for a human subject.

Administration may be repeated, e.g., twice, three or 4 times, e.g., with, 1, 2, 3, 4, 5, 6, 7, 10 or 14 days between administrations. Administration may also be repeated over extended periods of time, including once, twice, three times or four times a year.

### Examples

The invention is illustrated by the following examples, which describe in detail the synthesis of nanoparticles according to the present invention.

These examples should not be considered as limiting the scope of the invention, but as illustrating it.

### Example 1: Preparation of superparamagnetic iron oxide crystalline cores (SPIONs)

### a) Preparation of SPION Cores

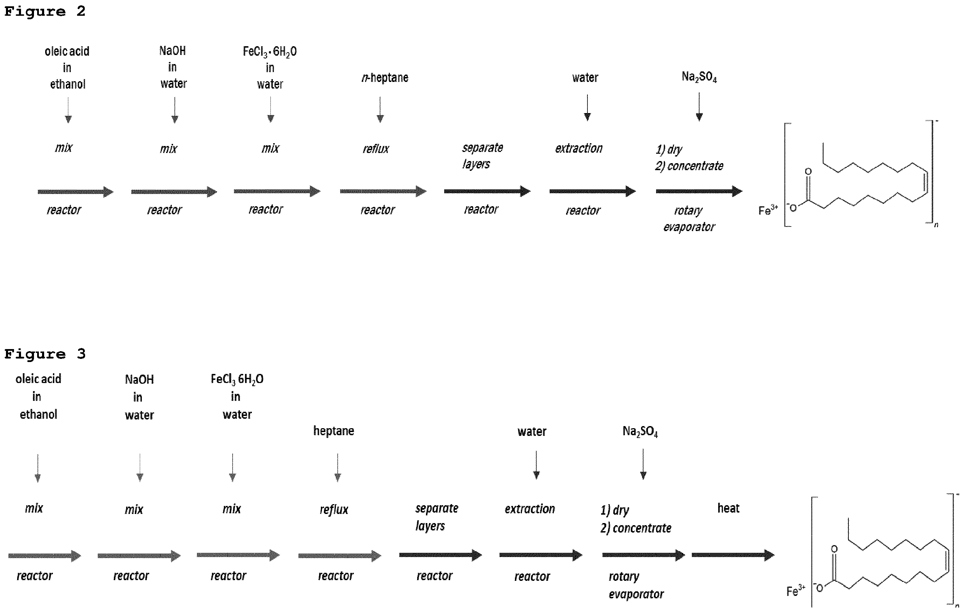

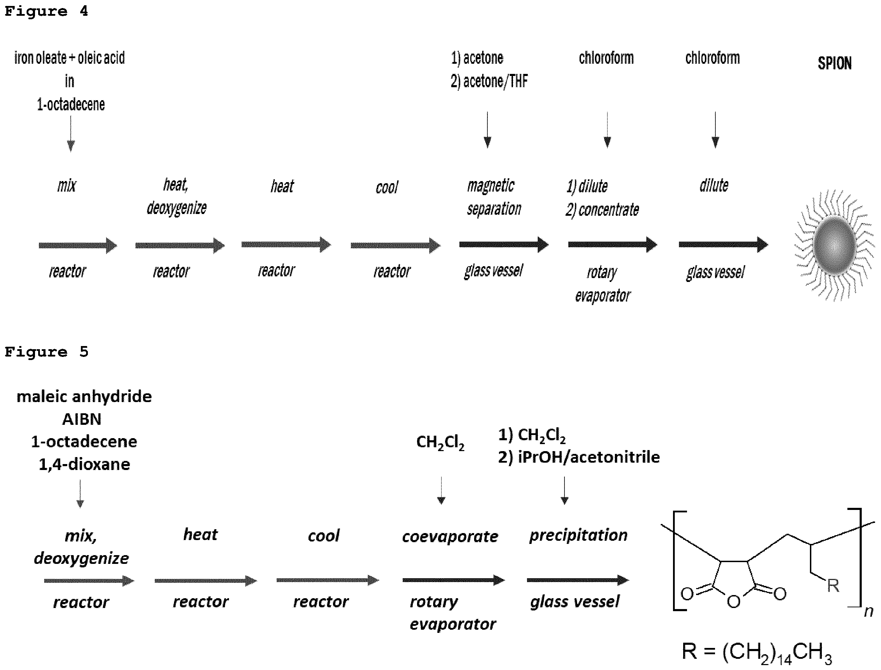

The method used for the synthesis of an iron oleate complex is schematically shown in Figure 2; an alternative method is shown in Figure 3. The synthesis of the SPIONS is schematically shown in Figure 4.

The iron oleate complex is prepared by mixing oleic acid in ethanol, sodium hydroxide and iron chloride in water under reflux with heptane at 70 °C. After reaction, the product is purified by several extractive washing steps in a reactor. The organic phase is dried over anhydrous sodium sulphate and then finally concentrated in a rotary evaporator. In the alternative method illustrated in Figure 3, the iron oleate complex is further heated for 24 hours at 80°C under vaccum conditions and the final product is stored at 5±3°C.

In a second step illustrated in Figure 4, the iron oleate complex was dissolved in 1-octadecene together with oleic acid at room temperature and stirred until complete dissolution. The solution was deoxygenated, dehydrated at 110 °C and then heated at 300 °C for the formation of iron oxide nanocrystals. After cooling down, the product was purified by several washing steps with acetone and tetrahydrofuran using magnetic separation. Purified SPIONs were then diluted in chloroform, concentrated in a rotary evaporator and finally diluted in chloroform for use in further manufacturing steps, as described below.

### b) Preparation of low molecular weight poly(maleic acid-alt-1-octadecene) (LM-PMAcOD)

The synthesis of low molecular weight poly(maleic acid-alt-1-octadecene) (LM-PMAcOD) was carried out in a two-step process schematically illustrated in Figures 5-8.

In a first step (Figure 5), a copolymerization of 1 octadecene and maleic anhydride, initiated by 2,2′-azobis(2 methylpropionitrile) (AIBN) in 1,4 dioxane, is carried out. The product is purified by co-evaporation with dichloromethane and precipitation with isopropanol and acetonitrile, providing low molecular weight poly(maleic anhydride-alt-1 octadecene), or LM-PMAOD, with number average molecular weight (Mn) 2500 4000 g/mol.

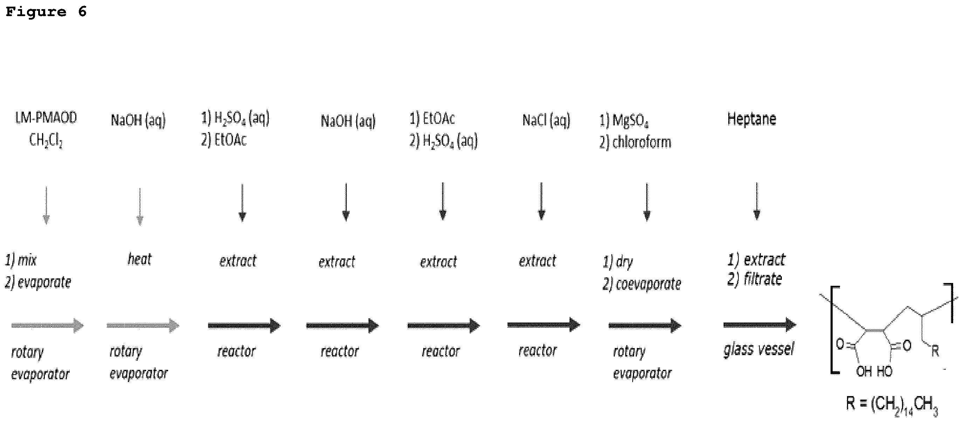

In a second step (Figure 6), LM-PMAOD is hydrolysed to poly(maleic acid-alt-1 octadecene) (LM-PMAcOD) in sodium hydroxide solution. Two acid-base extraction steps are carried out using H₂SO₄, ethylacetate, and NaOH for the purification of the product and to remove impurities such as residual 1 octadecene. The product is dried over magnesium sulphate, co-evaporated with chloroform and finally purified by solid-liquid extraction in heptane.

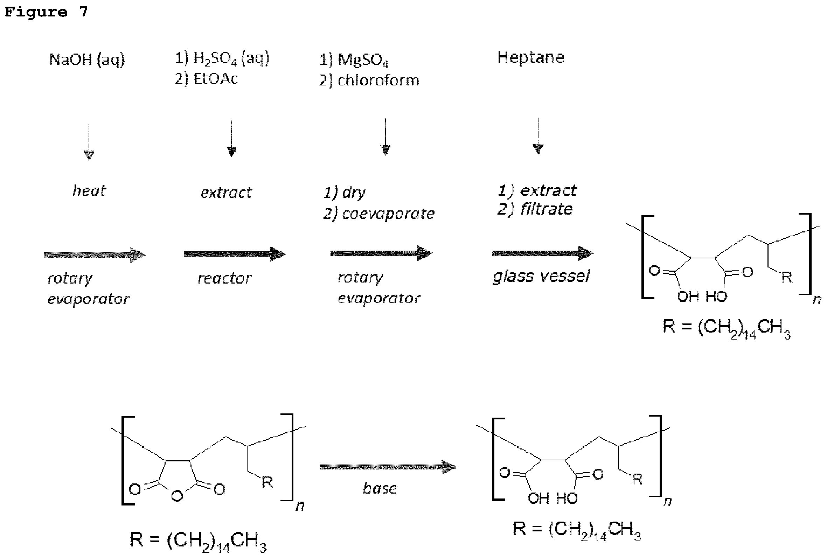

An alternative method is shown in Figure 7, which comprises the above hydrolysis step and only a single acid-base extraction step using H₂SO₄ and ethylacetate for the purification of the product. The other steps of the alternative method are the same as shown in Figure 6.

### c) Polymer coating of SPIONs

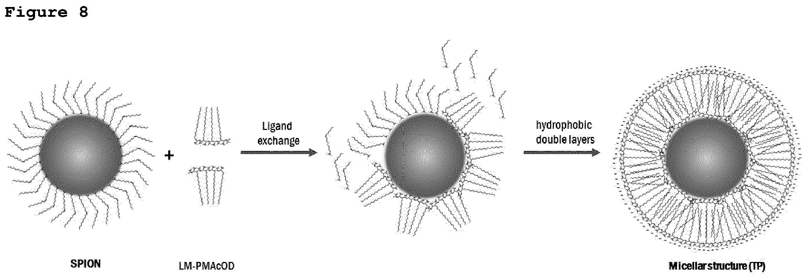

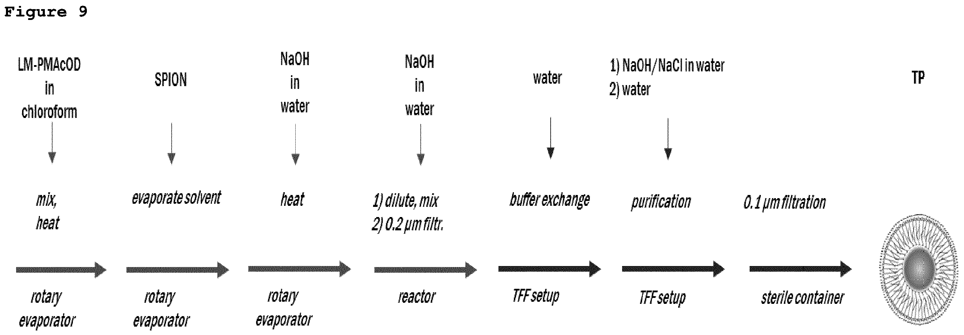

The polymer coating of the SPIONS is schematically shown in Figure 8. A flow chart of the PMAcOD-SPION particle synthesis is shown in Figure 9.

The micellar structure was formed by the arrangement of the amphiphilic polymer PMAcOD around the SPION core. Most of the oleate molecules present on the surface of the SPIONs were replaced by ligand exchange with PMAcOD units, whereby the hydrophobic polymer side chains form hydrophobic double layers constituting a negatively charged micellar structure. The charged carboxylate groups on the surface of the micelle act as anchor for the peptides.

For the coating procedure, the polymer and SPIONs were dissolved in chloroform. The solvent was removed via rotary evaporation until a thin film was formed. Sodium hydroxide solution was added and the flask was rotated at increased temperature and ambient pressure until a clear dark brown aqueous colloidal dispersion was formed. The dispersion was diluted in sodium hydroxide solution and filtered through a 0.2 µm-filter. Then, several washing steps with water and NaOH/NaCl in water were performed using tangential flow filtration (TFF) for the separation of low molecular weight components, followed by filtration through a 0.1 µm-filter to remove larger particles and aggregates as well as for sterilization of the PMAcOD-SPION dispersion. The final nanoparticle was dispersed in water (Figure 9).

### d) Peptides and peptide coupling

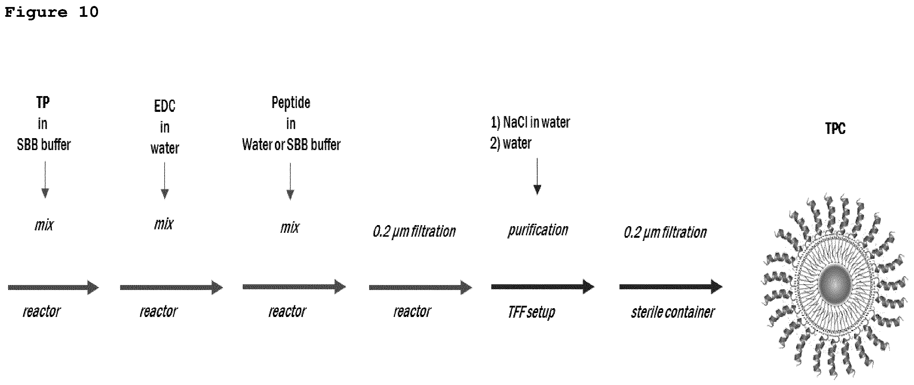

The peptide coupling and nanoparticle synthesis is schematically shown in Figure 10.

The synthesis of the peptides was accomplished via Fmoc chemistry from the C to N direction using solid phase peptide synthesis (SPPS). The alpha amino group of each amino acid was protected with a fluoren-9-ylmethoxycarbonyl (Fmoc) group, while side chain functional groups were also blocked with various appropriate protective groups.

The peptide was purified through preparative HPLC to reach purity requirements, and the counter ion TFA was replaced with chloride by using an appropriate solvent-buffer system. Finally, the purified peptide was lyophilized.

Characterization of the free peptide (starting materials) was performed by LC-MS. The molecular weight of the peptide was measured by multimode electrospray atmospheric pressure chemical ionization mass spectrometry.

| Sequence | Molecular formula | Monoisotopic Mass* | m/z [M+H]⁺ | m/z [M+H]²⁺ | m/z [M+H]³⁺ |
|---|---|---|---|---|---|
| LNSKIAFKIVSQEPA | C₇₅H₁₂₅N₁₉O₂₂ | 1643.9 | 1644.6 | 823.0 | 549.0 |
| TPMFLLSRNTGEVRT | C₇₄H₁₂₄N₂₂O₂₃S | 1720.9 | 1722.4 | 861.4 | 574.8 |
| NIKVKDVNDNFPMFR | C₈₂H₁₂₉N₂₃O₂₃S | 1835.9 | 1837.4 | 919.0 | 613.2 |
| REGIAFRPASKTFTV | C₇₆H₁₂₂N₂₂O₂₁ | 1678.9 | 1679.4 | 840.4 | 560.8 |

| | | | | | |
|---|---|---|---|---|---|
| * Monoisotopic mass, theoretical in Da | | | | | |

The peptide was coupled to the polymer surface using 1-ethyl-3-(3-dimethylaminopropyl)carbodiimide (EDC) chemistry in boric acid/sodium tetraborate decahydrate (SBB) buffer. The resulting TPC preparation was then filtered and purified by TFF purification. The final nanoparticle was dispersed in water, filtered through a 0.2 µm-filter and collected into a sterile container.

### Example 2: Treatment efficacy of composition comprising three versus 4 peptide conjugates

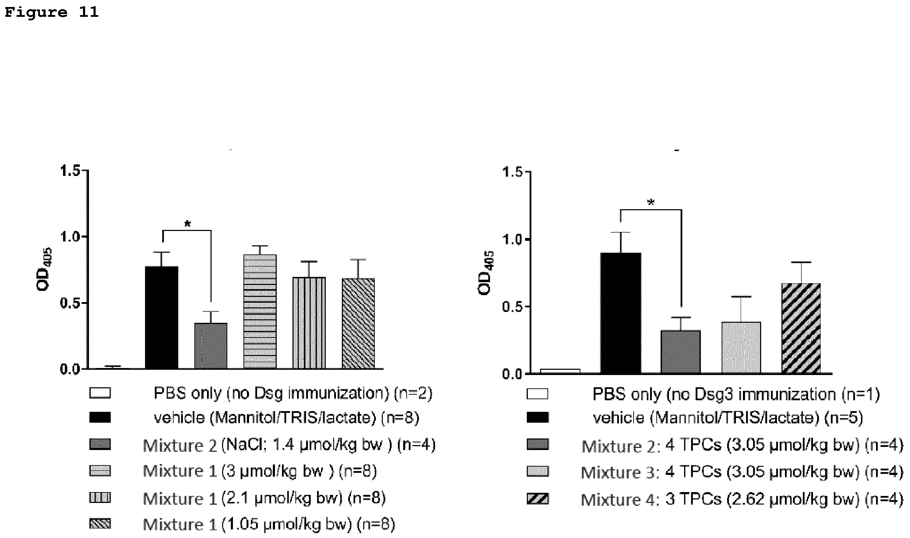

In this experiment, treatment efficacy of different compositions comprising different nanoparticles was compared. Briefly compositions comprising three different peptides were compared to compositions comprising nanoparticles comprising four different peptides. The results are shown in Figure 11.

The following different mixtures tested were:

| | |
|---|---|
| Mixture 1: | equimolar mixture of three different nanoparticles, each coupled to one of peptides with SEQ ID NO:1, 2 and 4, tested in three different concentrations (3, 2.1, 1.05 µmol/kg) |
| Mixture 2: | non-equimolar mixture of four different nanoparticles, each coupled to one of peptides with SEQ ID NO:1-4. (3.05 µmol/kg) |
| Mixture 3: | equimolar mixture of four different nanoparticles, each coupled to one of peptides with SEQ ID NO:1-4. (3.05 µmol/kg) |
| Mixture 4: | non-equimolar mixture of three TPC-Dsg3-peptides, tested in three different nanoparticles, each coupled to one of peptides with SEQ ID NO:1, 2 and 4 (2.62 µmol/kg) |
| Control: | PBS buffer |

The different mixtures used in the Studies PoC5 and 6 were prepared in mannitol-Tris-lactic acid buffer (5% mannitol + 5mM Tris + 6mM L-lactic acid). Only in PoC5, mixture 2 was formulated in 100mM NaCl solution.

For induction of anti-Dsg3 antibodies, 8-12-week-old female and male HLA-DRB1*04:02-transgenic mice were immunized by intraperitoneal injection of recombinant Dsg3 protein in alum on Day 0, followed by a second immunization on Day 14, as described elsewhere (Eming et al., J Immunol. 193, 4391-9 (2014)). This Dsg3-immunization protocol induced a detectable increase in antibody titers from Day 21 on, with robust IgG titers on Day 28.

The different mixtures of nanoparticles as well as the relevant controls were injected into the tail veins of HLA-transgenic mice one day before each of the two immunizations with Dsg3, thereby generating a situation that resembled a preventive approach.

Experiments were completed 28 days after immunization. As primary readout of TPC treatment efficacy, the generation of anti-Dsg3 antibodies was monitored during the course of the experiments, by anti-Dsg3 ELISA as described in Eming et al., J Immunol. 193, 4391-9 (2014).

For this purpose, serum samples were taken at baseline before immunization (e.g. on Day -2) and on Days 12, 21 and 28 after immunization. Statistical analysis of Dsg3-IgG ELISA values was performed by using the Mann-Whitney test after performing the D'Agostino & Pearson normality test. Data are shown as mean with SEM.

Those immunized mice receiving only buffer (vehicle) generated high levels of anti-Dsg3 IgG after 28 days and served as control.

As shown in the left graph of Figure 10, in contrast to a significant reduction in anti-Dsg3-IgG titers by mixture 2, no effect was observed with mixture 1 at any of the three concentrations tested.

Additionally, as shown in the right graph of Figure 10

## Claims

1. A pharmaceutical composition comprising nanoparticles, wherein the nanoparticles each comprise
a) a micelle comprising an amphiphilic polymer, and
b) at least one peptide,
wherein the pharmaceutical composition comprises peptides comprising SEQ ID NO:1-4.

2. The pharmaceutical composition of claim 1, wherein the nanoparticles further comprise a solid hydrophobic core which is at least partially coated by the micelle, wherein the core comprises a traceable inorganic material selected from the group comprising iron oxide, CdSe/CdS/ZnS, silver and gold.

3. The pharmaceutical composition of any of the preceding claims, wherein the at least one peptide is associated with the outside of the micelle.

4. The pharmaceutical composition of any of the preceding claims, wherein each peptide consists of one of the sequences SEQ 1-4.

5. The pharmaceutical composition of any of the preceding claims comprising at least 4 different types of nanoparticles, wherein the different types of nanoparticles differ among each other in the peptide sequence, and wherein a first type of nanoparticles comprises peptides having SEQ ID NO:1, a second type of nanoparticles comprises peptides having SEQ ID NO:2, a type of nanoparticles comprises peptides having SEQ ID NO:3 and a forth type of nanoparticles comprises peptides having SEQ ID NO:4.

6. The pharmaceutical composition of claim 5, wherein each type of nanoparticle is present in equimolar amounts.

7. The pharmaceutical composition of any of the preceding claims, wherein the pharmaceutical composition comprises only peptides having sequences SEQ ID NO:1-4.

8. The pharmaceutical composition of any of the preceding claims, wherein the amphiphilic polymer has a number average molecular weight (Mn) of 20,000 g/mol or less, preferably 10,000 g/mol or less, most preferably 6,000 to 1,000 g/mol.

9. The pharmaceutical composition of the preceding claims, wherein the amphiphilic polymer comprises the following building block 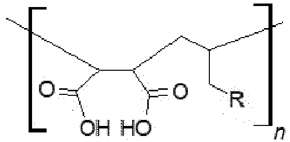 wherein R is a hydrocarbyl group or a substituted hydrocarbyl group, preferably R is a C₄ to C₂₂ alkyl group, preferably C₈ to C₂₀ alkyl group.

10. The pharmaceutical composition of any of the preceding claims, wherein the amphiphilic polymer is selected from the group comprising poly(maleic acid-alt-1-octadecene), poly(maleic acid-*alt*-1-dodecene) and poly(maleic acid-alt-1-tetradecene), preferably the polymer is poly(maleic acid-alt-1-octadecene), and the number average molecular weight of the polymer is from 6,000 to 1,000 g/mol.

11. The pharmaceutical composition of any of the preceding claims, wherein each peptide is covalently linked to the micelle or non-covalently associated.

12. The pharmaceutical composition of any of the preceding claims further comprising an aqueous buffer, wherein the aqueous buffer preferably comprises at least one sugar, at least one primary amine and at least one aminoacid.

13. The pharmaceutical composition of claim 12, wherein the buffer contains D-mannitol, Tris(hydroxymethyl)aminomethane and L-lactic acid.

14. The pharmaceutical composition of any of the preceding claims, wherein each nanoparticle comprises an iron oxide core and a micelle comprising poly(maleic acid-alt-1-octadecene) having a number average molecular weight of 6,000 to 1,000 g/mol.

15. The pharmaceutical composition of any of the preceding claims for use in the treatment of Pemphigus vulgaris.
